# EUROPEAN PATENT SPECIFICATION

(11) **EP 0 791 601 B1**
(45) Date of publication and mention of the grant of the patent: **27.04.2005**
(21) Application number: 97102731.3
(22) Date of filing: 20.02.1997
(51) Int. Cl.: C07K 14/47, A61K 38/17, C07K 14/51, A23K 1/16, A23L 1/305, A61K 38/18, A23K 1/18

(54) **An agent promoting bone formation and inhibiting bone resorption**
Mittel zur Verbesserung der Knochenbildung und zur Inhibierung der Knochenresorption
Agent améliorant la formation d'os et inhibitant la résorption osseuse

(30) Priority: 23.02.1996 JP 6198796
(43) Date of publication of application: 27.08.1997
(73) Proprietor: SNOW BRAND MILK PRODUCTS CO., LTD., Sapporo-shi, Hokkaido 065 (JP)
(72) Inventor: Takada, Yukihiro, Kawagoe-shi, Saitama (JP); Yamamura, Junichi, Kawagoe-shi, Saitama (JP); Goto, Masaaki, Shimotsuga-gun, Tochigi (JP); Aoe, Seiichiro, Sayama-shi, Saitama (JP)
(74) Representative: Boeters, Hans Dietrich, Dr.

(56) References cited:
- EP-A- 0 573 668
- MAJUMDAR ET AL.: "Sequence of human HMG2 cDNA" NUCLEIC ACIDS RESEARCH, vol. 19, no. 23, 1991, page 6643 XP002078449
- ADACHI ET AL.: "Efficient large-scale purification of non-histone chromosomal proteins HMG1 and HMG2 by using polybuffer-exchanger PBE94" JOURNAL OF CHROMATOGRAPHY, vol. 530, 1990, pages 39-46, XP002078450
- MERENMIES ET AL.: "30-kDa heparin-binding protein of brain (amphoterin) involved in neurite outgrowth" THE JOURNAL OF BIOLOGICAL CHEMISTRY, vol. 266, no. 25, 5 September 1991, pages 16722-16729, XP002078451
- STERNER ET AL.: "Discrete proteolytic cleavage of high mobility group proteins" BIOCHEMICAL BIOPHYSICAL RESEARCH COMMUNICATIONS, vol. 89, no. 1, 12 July 1979, pages 129-133, XP002078452
- PENTECOST ET AL.: "Isolation and partial sequence of bovine cDNA clones for the high-mobility-group protein (HMG-1)" BIOSCIENCE REPORTS, vol. 4, 1984, pages 49-57, XP002078453
- SHAKOORI ET AL.: "Differential expression of the chromosomal high mobility group proteins 14 and 17 during the onset of differentiation in mammalian osteoblasts and promyelocytic leukemia cells" JOURNAL OF CELLULAR BIOCHEMISTRY, vol. 51, 1993, pages 479-487, XP002078454
- DATABASE WPI Week 9232 Derwent Publications Ltd., London, GB; AN 92-265574 XP002078455 & JP 04 183371 A (SNOW BRAND MILK PROD. CO. LTD.), 30 June 1992

## Description

### FIELD OF THE INVENTION

The present invention relates to the use of an agent comprising a protein having a specific N-terminal amino acid sequence such as HMG (high-mobility-group) protein and Amphoterin etc. and/or degrada-tion product thereof as an effective ingredient for the preparation of a medicament for promoting bone formation or inhibiting bone resorption. Further, the present invention also relates to the use of said agent in combination with a drink or food.

### BACKGROUND OF THE INVENTION

Accompanying with the prolongation of human life span, the incidence of metabolic bone diseases such as osteoporosis, bone fracture, bone pain etc., recently increased. In bone tissue, bone formation and bone resorption are always occurring. While the balance of bone formation and bone resorption is kept in one's youth, bone resorption exceeds bone formation due to various causes as one's age increases (uncoupling). And when bone resorption prolongs for a long duration, bone tissue becomes fragile, which causes metabolic bone diseases such as osteoporosis, bone fracture, bone pain, etc., are expected to be prevented.

As conventional methods of preventing or treating metabolic bone diseases by inhibiting uncoupling, (1) calcium supplemented diets, (2) light exercise, (3) sunbathing, (4) medicinal therapy, etc., are exemplified. As for calcium supplemented diets, calcium salts such as calcium carbonate, calcium phosphate, etc., and naturally occurring calcium-containing preparation, such as bovine bone powder, egg shell, fish bone powder, etc. are used. They are, however, not necessarily good for oral intake. As light exercises, jogging or walking may be recommended. However, they are troublesome to a person who becomes weak and quite difficult to an immobilized aged person. Sunbathing is believed to be good for supplement of active form of vitamin D₃ but is not sufficient. As medicinal therapy, 1 α-hydroxyvitamin D₃ and/or calcitonin may be used and they are known to be effective for treating osteoporosis. However, they are medicines themselves and can not be used as food sources.

On the other hand, the present inventors have investigated to find out a factor strengthening bone and inhibiting bone resorption present in whey proteins for obtaining substances having an action of strengthening bone and/or inhibiting bone resorption which is useful for food sources. Eventually, we found out that protein and/or peptide mixture obtained by removing salt from water soluble fraction of whey protein by treatment with reverse osmotic membrane and/or by electrophoresis had an action of strengthening bone (Japanese published unexamined patent application No. 183371/1992). Further, we found that a fraction obtained from liquid solution of this protein and peptide mixture by ethanol treatment, heating, addition of salt and ultrafiltration treatment had an action of promoting the osteoblastic proliferation and strengthening bone (Japanese published unexamined patent application No. 176715/1993, Japanese published unexamined patent application 320066/1993). In addition, we also found that basic protein present in a very small amount in milk had an action of promoting the osteoblastic proliferation, strengthening bone and inhibiting bone resorption (Japanese patent application No. 207509/1995). The present inventors tried to isolate and purify an active component having an action of promoting the osteoblastic proliferation and inhibiting bone resorption from milk. Finally, we isolated such a substance having an action as described above, purified and identified it as HMG protein or Amphoterin. These proteins were found not only in milk but also in mammalian thymus, brain etc., and also found to be distributed widely in the whole body. We also found that the HMG protein and Amphoterin isolated from the above tissue had the same action as those isolated from milk. Since the amino acid sequence at N-terminus in these HMG proteins and Amphoterins is the same (Bio-science Reports, vol.4, pp49-57, 1984, and J. Biol. Chem., vol. 266, pp 16722-16729, 1991), it is expected that a protein having this N-terminal amino acid sequence has the action described above. Further, the present inventors found that degradation products thereof had also the same action.

### SUMMARY OF THE INVENTION

Accordingly, the present invention discloses the use of an agent for the preparation of a medicament for promoting bone formation or inhibiting bone resorption comprising protein and/or degradation product thereof whose amino acid sequence at N-terminus is Gly-Lys-Gly-Asp-Pro-Lys-Lys-Pro-Arg-Gly-Lys-Met-Ser-Ser-Tyr-Ala-Phe-Phe-Val-Gln-Thr-Cys-Arg-Glu-Glu-His-Lys-Lys-Lys-His as an effective ingredient. Moreover, the present invention discloses the use of said agent in combination with a drink or food.

### DETAILED DESCRIPTION OF THE INVENTION AND PREFERRED EMBODIMENTS

The present invention uses an agent as an effective ingredient for the preparation of a medicament for promoting bone formation and inhibiting bone resorption which is a protein having the following common amino acid sequence at the N-terminus thereof:

Gly-Lys-Gly-Asp-Pro-Lys-Lys-Pro-Arg-Gly-Lys-Met-Ser-Ser-Tyr-Ala-
Phe-Phe-Val-Gln-Thr-Cys-Arg-Glu-Glu-His-Lys-Lys-Lys-His-

These proteins include HMG protein and Amphoterin and have the same N-terminal amino acid sequence regardless of the origin thereof. In the present invention, degradation products thereof can be also used as an effective ingredient.

HMG protein which is used as an effective ingredient in the present invention is a sort of non-histone protein and comprises a large amount of basic amino acid and acidic amino acid as composing amino acid. And it has a basic N-terminus and acidic C-terminus, which is non-symmetrical. The binding modality between HMG protein and DNA is ionic. It is known that there is an interaction between basic amino acid and phosphate of DNA. This HMG protein had been found in the whole tissue of higher class of biological organisms so far and known to have similarity to a certain extent to histone (Kaplan, D.J, Duncan,C.H., Nucleic Acid Res., vol.16, p. 10375, 1988).

The HMG protein can be obtained by various kinds of treatment of skim milk which was isolated from milk such as cow milk, breast milk, goat milk and sheep milk,etc.,and centrifuged, such as heating, addition of salt, ethanol treatment, ion exchange chromatography, gel filtration chromatography, other kinds of chromatography and ultrafiltration etc.

Alternatively, Amphoterin which is used as an effective ingredient in the present invention is 30 kDa of heparin binding protein and has a property to bind with cells in neuron system strongly. It was also said to be a substance relating to cell adhesion and/or dendrite of cell and to play an important roll for development of brain.

Amphoterin can be obtained, like the HMG protein, by various kinds of treatment of skim milk which was isolated from milk such as cow milk, breast milk, goat milk and sheep milk,etc.,and centrifuged, such as heating, addition of salt, ethanol treatment, ion exchange chromatography, gel filtration chromatography, other kinds of chromatography and ultrafiltration etc.

The degradation products of the HMG protein and Amphoterin which are used as an effective ingredient of the present invention are a peptide mixture obtained by degradation of the HMG protein or Amphoterin with trypsin, chymotripsin, pepsin, papain, kalikrein, cathepsin, thermolysin and V8 protease etc. whose molecular weight was 100-20000 Da (by HPLC gel filtration chromatography).

The present invention uses an agent for the preparation of a medicament for promoting bone formation and inhibiting bone resorption which comprises a protein having a specific N-terminus, such as HMG protein or Amphoterin, or degradation products thereof as an effective ingredient. The protein having a specific N-terminus or degradation products thereof can be combined with a drink or food, such as milk, milk drinks, coffee drinks, juice, jelly, cracker, bread, noodle or sausage etc., or can be used for a medicine as tablets, or powders etc. Further, combining said agent with a calcium agent which shows good calcium absorptivity, such as calcium chloride, calcium carbonate, calcium lactate, egg shell, calcium derived from milk, etc., the action thereof promoting bone formation can be augmented.

The agent promoting bone formation and inhibiting bone resorption used according to the present invention can be taken several times, separately with a dose of 100 mg - 10 mg per day in an adult person. By using the agent for promoting bone formation and inhibiting bone resorption according to the present invention, metabolic bone diseases such as osteoporosis can be prevented or improved.

Alternatively, these effective ingredients can be combined with feeds so that bone formation can be promoted and/or bone resorption can be inhibited in livestock or poultry.

As described above, the agent used for promoting bone formation or inhibiting bone resorption according to the present invention comprises a protein having a specific N-terminal amino acid sequence or degradation product thereof, such as HMG protein or Amphoterin, as an effective ingredient and can be used for prevention and/or improvement of various kinds of bone metabolic dieaeses such as osteo-porosis. Further, foods or drinks comprising these proteins or degradation products thereof can prevent and/or improve these bone metabolic diseases in the same manner as the above.

Then, the present invention will be described by examples or test examples. However, the scope of the present invention will not be limited by those examples.

### Example 1

A column packed with sulfonated Chitopearl (Fuji-boseki) was washed with deionized water and with sufficient volume of cationized water. On the cation exchange resin, 300 l of skim milk was applied and the resin was washed sufficiently with deionized water. Basic protein adsorbed thereto was eluted with linear gradient of 0.1-1M NaCl in 0.02 M carbonate buffer solution and recovered. Then, the eluted fraction comprising HMG protein was, further, loaded on a S-Sephrose column equilibrated with 0.1 M phosphate buffer solution (pH 6.5) and eluted with linear gradient of 0.1-1.0 M NaCl. The obtained fraction was heated at 90 C for 10 min. and centrifuged to remove a precipitate therefrom. Further, this fraction was loaded on a Mono Q ion exchange column equilibrated with 0.1 M phosphate buffer solution (pH 6.5) and eluted with linear gradient of 0.1-1.0 M NaCl. Then, the fraction was loaded on a Sepharose 12 gel filtration column and fractionated. Finally, the fraction was purified by high performance liquid chromatography using C4 reversed phase chromatography to give 135 mg of HMG-protein.

### Example 2

Preparation of HMG protein from porcine thymus was carried out according to the method of Y. Adachi (J. Chromatography, 530, 39-46 (1990).

Excised thymus chromatin (253g) was homogenized in 0.35 M NaCl with a potter-type homogenizer and centrifuged (5000 x g, 20 min.), which was dialyzed against 10 mM phosphate buffer solution (pH 7.8) overnight. The dialysate was loaded on a PBB-94 column equilibrated with 10 mM phosphate buffer solution (pH 7.8) and eluted with linear gradient of 0.1-1.0 M NaCl to yield 25 mg of HMG-protein.

### Example 3

Amphoterin was prepared according to the method of R.Heikki (Heikki, R., Riitta, P., J. Biol. Chem., vol. 262, pp.16625-16635, 1987). That is, 112.7 g of brain excised from SD rats was homogenized in 100 ml of ice-cooled PBS (137 mM sodium chloride, 27 mM potassium chloride, 8.1 mM disodium hydrogenphosphate, 1.5 mM potassium dihydrogenphosphate, pH 7.4) and centrifuged (100,000 x g, 1 hour) to recover a precipitate. Then, this precipitate was dissolved in 50 mM octyl glucoside containing 1 mM PMSF (phenylmethylsulfonyl fluoride) and 5 mM EDTA, stirred for 1 hour and centrifuged (100,000 x g, 1 hour) to remove a precipitate. The supernatant was loaded on a Heparine-Sepharose column and eluted with linear gradient of 2 M sodium chloride, followed by Superose 12 gel filtration chromatography and lyophilization to yield 12 mg of Amphoterin.

### Example 4

HMG protein (50 mg) obtained in example 1 was suspended in 10 ml of water and trypsin was added so that the final concentration would be 0.01 %. Enzymatic degradation was carried out at 37°C for 1 hour and stopped by deactivating the enzyme at 90°C for 5 min., followed by lyophilization to yield 43 mg of HMG protein degradation product. The molecular weight of this degradation product was determined to be 100-18,000 Da by HPLC gel filtration chromatography (Tosoh).

### Example 5

Amphoterin (5 mg) obtained in example 3 was suspended in 10 ml of water and pancreatin was added thereto so that the final concentration thereof would be 0.001 %. Enzymatic degradation was carried out at 37°C for 5 hours and stopped by deactivating the enzyme at 90°C for 5 min., followed by lyophilization to yield 4.3 mg of Amphoterin degradation product. The molecular weight of this degradation product was determined to be 200-20,000 Da by HPLC gel filtration chromatography.

### Test example 1

Substances obtained in example 1-5 were investigated with respect to action of promoting osteoblastic proliferation. That is, 2 x 10⁴ cells/ml of mouse osteoblast cell line MC3T3-E1 in α-modified minimum essential medium (α-MEM) medium containing 10 % bovine fetal serum (Flow laboratories) was inoculated in each well of 96-well plate and cultured at 37°C for test culture. Then, the medium was changed into α-MEM which did not contain bovine fetal serum, to which fraction obtained in example 1-5 was added so that the final concentration thereof would be 10 µg/ml and cultured at 37°C for 18 hours. Then, 2 hours after 0.02 MBq of ³H-thymidine was added thereto, cells were collected on a filter paper by cell harvester, radioactivity incorporated into cells was counted by a liquid scintillation counter for determination of osteoblastic proliferative activity. As control group, culture without any addition were used. The proliferative activity in each case was calculated (%) by defining 100 % as that in the case of culture without any addition and the results were represented in Table 1.

**Table 1**

| Fraction | Osteoblastic proliferative activity |
|---|---|
| Example 1 | 189 ± 25 (%) |
| Example 2 | 183 ± 29 |
| Example 3 | 140 ± 19 |
| Example 4 | 153 ± 15 |
| Example 5 | 143 ± 16 |

Comparing with proliferative activity of the non-added group, that of any group with addition of substance obtained in example 1-5 was higher, which demonstrated that they had action of promoting osteoblastic proliferation. And the similar results were obtained in the case of test culture using another osteoblast cell line UMR.

### Test example 2

Substances obtained in example 1-5 were investigated with respect to inhibiting action thereof on bone resorption. Long bones were extirpated from 10-20 days old ICR mica and the whole bone marrowcells comprising osteoclast were obtained by depriving soft tissue from the bones and mincing the bones in α-MEM containing 5 % bovine fetal serum mechanically. About 2 x 10⁶ of these cells in α-MEM containing 5 % bovine fetal serum were placed on a piece of dentinum. Two hours after then, a test sample in α-MEM containing 5 % bovine fetal serum was added thereto so that the final concentration thereof would be 10 µg/ml, which was cultured for 5 days and bone osteoblastic resorptive activity was investigated. Analysis of bone resorption was carried out by depriving cells from a piece of dentinum after cultivation thereof, staining them with Hematoxylin dye and counting the number of bone resorptive pit by morphometrical analysis with PIAS LA-555. As control group, culture without any addition was used. Bone resorptive activity of each case was calculated by defining 100 % as that in the case of non-added group (%). The results were represented in Table 2.

**Table 2**

| | Bone resorptive activity |
|---|---|
| Example 1 | 70.7 ± 5.7 |
| Example 2 | 79.9 ± 5.7 |
| Example 3 | 77.9 ± 8.6 |
| Example 4 | 75.4 ± 8.6 |
| Example 5 | 85.4 ± 8.6 |

Comparing with bone resorptive activity of non-added group, that of any group with addition of substance obtained in example 1-5 was inhibited more. Therefore, it was found that they had action of inhibiting bone resorption.

### Example 6

A drink having action of promoting bone formation and inhibiting bone resorption was prepared by mixing raw materials represented in Table 3, packing it in a container and sterilizing it by heating.

**Table 3**

| | |
|---|---|
| Mixed isomerized sugar | 15.0 (weight %) |
| Fruit juice | 10.0 |
| Citric acid | 0.5 |
| Substance obtained in example 3 | 0.0005 |
| Flavor | 0.1 |
| Calcium | 0.5 |
| Water | 73.9 |

### Example 7

A tablet having action of promoting bone formation and inhibiting bone resorption was prepared by mixing raw materials represented in Table 4 and formulating it under pressure.

**Table 4**

| | |
|---|---|
| Crystalline glucose hydrate | 93.5 (weight %) |
| Substance obtained example 2 | 0.05 |
| Calcium | 5.0 |
| Sugar ester | 1.0 |
| Flavor | 0.5 |

### Example 8

A cracker having action of promoting bone formation and inhibiting bone resorption was prepared by mixing raw materials represented in Table 5, making dough, formulating and baking it.

**Table 5**

| | |
|---|---|
| Wheat powder | 50.0 (weight %) |
| Sugar | 20.0 |
| Sodium chloride | 0.5 |
| Margarine | 12.5 |
| Egg | 12.1 |
| Water | 3.7 |
| Sodium bicarbonate | 0.1 |
| Ammonium bicarbonate | 0.2 |
| Calcium carbonate | 0.1 |
| Substance obtained in example 2 | 0.005 |

### Example 9

A jelly having action of promoting bone formation and inhibiting bone resorption was prepared by mixing raw materials represented in Table 6, packing it in container and sterilizing it by heating.

**Table 6**

| | |
|---|---|
| Fructose | 20.0 (weight %) |
| Granulated sugar | 15.0 |
| Miller jelly | 5.0 |
| Agar | 1.0 |
| Substance obtained in example 4 | 0.0005 |
| Flavor | 0.11 |
| Calcium | 0.1 |
| Water | 58.39 |

### Example 10

A processed cheese having action of promoting bone formation and inhibiting bone resorption was prepared by mixing raw materials represented in Table 7 and emulsifying it at 85°C.

**Table 7**

| | |
|---|---|
| Gouda cheese | 43.0 (weight %) |
| Cheddar cheese | 43.5 |
| Sodium citrate | 2.0 |
| Substance obtained in example 1 | 0.005 |
| Milk-derived calcium | 1.0 |
| Water | 10.5 |

### Example 11

After sterilizing 12 weight % reducing skim milk at 90°C for 20 min., Lactobacillus acidophilus and Streptococcus thermophilus were inoculated thereon to give 2 kinds of starter culture, which were mixed in the same amount. A yogurt having action of promoting bone formation and inhibiting bone resorption was prepared by mixing raw materials represented in Table 8 and fermenting it.

**Table 8**

| | |
|---|---|
| Yogurt mix | 97.0 (weight %) |
| Starter culture | 3.0 |
| Substance obtained in example 1 | 0.0005 |

### Example 12

A dry milk for infant having action of promoting bone formation and inhibiting bone resorption was prepared by mixing raw materials represented in Table 9.

**Table 9**

| | |
|---|---|
| Skim milk | 75.61 (weight %) |
| Whey protein condensate | 2.36 |
| Lactose | 13.86 |
| Mineral mix | 0.32 |
| Water soluble vitamin mix | 0.32 |
| Fat containing fat-soluble vitamin | 7.53 |
| Substance obtained in example 1 | 0.001 |

### Example 13

A feed for dog having action of promoting bone formation and inhibiting bone resorption was prepared by mixing raw materials represented in Table 10.

**Table 10**

| | |
|---|---|
| Soy bean cake | 12.0 |
| Skim milk powder | 14.0 |
| Soy bean oil | 4.0 |
| Corn oil | 2.0 |
| Palm oil . | 28.0 |
| Corn starch | 15.0 |
| Wheat powder | 9.0 |
| Wheat bran | 2.0 |
| Vitamin mix | 9.0 |
| Mineral mix | 2.0 |
| Cellulose | 3.0 |
| Substance obtained in example 1 | 0.001 |

## Claims

1. Use of an agent comprising a protein and/or a degradation product thereof whose amino acid sequence at the N-terminus is Gly-Lys-Gly-Asp-Pro-Lys-Lys-Pro-Arg-Gly-Lys-Met-Ser-Ser-Tyr-Ala-Phe-Phe-Val-Gln-Thr-Cys-Arg-Glu-Glu-His-Lys-Lys-Lys-His as an effective ingredient for the preparation of a medicament for promoting bone formation or inhibiting bone resorption.

2. Use according to claim 1, wherein the protein whose amino acid sequence at the N-terminus is Gly-Lys-Gly-Asp-Pro-Lys-Lys-Pro-Arg-Gly-Lys-Met-Ser-Ser-Tyr-Ala-Phe-Phe-Val-Gln-Thr-Cys-Arg-Glu-Glu-His-Lys-Lys-Lys-His is the HMG (high-mobility-group) protein.

3. Use according to claim 1, wherein the protein whose amino acid sequence at the N-terminus is Gly-Lys-Gly-Asp-Pro-Lys-Lys-Pro-Arg-Gly-Lys-Met-Ser-Ser-Tyr-Ala-Phe-Phe-Val-Gln-Thr-Cys-Arg-Glu-Glu-His-Lys-Lys-Lys-His is Amphoterin.

4. Use according to claim 1, wherein said protein and/or degradation product thereof is combined with calcium.

5. Use according to any one of claims 1-4, wherein said protein degradation product is a peptide mixture having molecular weight of 100-20,000 Da (determined by HPLC gel permeation chromatography) obtained from limited degradation of HMG protein or Amphoterin by protease.

6. Use according to any one of the preceeding claims, wherein the agent is combined with a drink or food.

## Patentansprüche

1. Verwendung eines Mittels, umfassend als wirksamen Bestandteil ein Protein und/oder ein Abbauprodukt desselben, dessen N-terminale Aminosäuresequenz Gly-Lys-Gly-Asp-Pro-Lys-Lys-Pro-Arg-Gly-Lys-Met-Ser-Ser-Tyr-Ala-Phe-Phe-Val-Gln-Thr-Cys-Arg-Glu-Glu-His-Lys-Lys-Lys-His ist, für die Herstellung eines Medikaments zur Förderung der Knochenbildung oder Inhibierung der Knochenresorption.

2. Verwendung nach Anspruch 1, wobei das Protein, dessen N-terminale Aminosäuresequenz Gly-Lys-Gly-Asp-Pro-Lys-Lys-Pro-Arg-Gly-Lys-Met-Ser-Ser-Tyr-Ala-Phe-Phe-Val-Gln-Thr-Cys-Arg-Glu-Glu-His-Lys-Lys-Lys-His ist, das HMG (high-mobility-group) Protein ist.

3. Verwendung nach Anspruch 1, wobei das Protein, dessen N-terminale Aminosäuresequenz Gly-Lys-Gly-Asp-Pro-Lys-Lys-Pro-Arg-Gly-Lys-Met-Ser-Ser-Tyr-Ala-Phe-Phe-Val-Gln-Thr-Cys-Arg-Glu-Glu-His-Lys-Lys-Lys-His ist, Amphoterin ist.

4. Verwendung nach Anspruch 1, wobei das Protein und/oder das Abbauprodukt desselben mit Calcium kombiniert wird.

5. Verwendung nach einem der Ansprüche 1-4, wobei das Abbauprodukt des Proteins eine Peptidmischung mit einem Molekulargewicht von 100-20.000 Da (bestimmt durch HPLC-Gelpermeationschromatographie) ist, erhalten aus dem begrenzten Abbau von HMG Protein oder Amphoterin durch Protease.

6. Verwendung nach einem der vorhergehenden Ansprüche, wobei das Mittel mit einem Getränk oder Nahrungsmittel kombiniert wird.

## Revendications

1. Utilisation d'un agent comprenant une protéine et/ou un produit de dégradation de celle-ci dont la séquence d'acides aminés à l'extrémité N terminale est Gly-Lys-Gly-Asp-Pro-Lys-Lys-Pro-Arg-Gly-Lys-Met-Ser-Ser-Tyr-Ala-Phe-Phe-Val-Gln-Thr-Cys-Arg-Glu-Glu-His-Lys-Lys-Lys-His en tant qu'ingrédient efficace pour la préparation d'un médicament pour favoriser la formation d'os ou inhiber la résorption osseuse.

2. Utilisation selon la revendication 1, dans laquelle la protéine dont la séquence d'acides aminés à l'extrémité N terminale est Gly-Lys-Gly-Asp-Pro-Lys-Lys-Pro-Arg-Gly-Lys-Met-Ser-Ser-Tyr-Ala-Phe-Phe-Val-Gln-Thr-Cys-Arg-Glu-Glu-His-Lys-Lys-Lys-His est la protéine HMG (groupe à mobilité élevée).

3. Utilisation selon la revendication 1, dans laquelle la protéine dont la séquence d'acides aminés à l'extrémité N terminale est Gly-Lys-Gly-Asp-Pro-Lys-Lys-Pro-Arg-Gly-Lys-Met-Ser-Ser-Tyr-Ala-Phe-Phe-Val-Gln-Thr-Cys-Arg-Glu-Glu-His-Lys-Lys-Lys-His est l'amphotérine.

4. Utilisation selon la revendication 1, dans laquelle ladite protéine et/ou son produit de dégradation est combiné avec le calcium.

5. Utilisation selon l'une quelconque des revendications 1 à 4, dans laquelle ledit produit de dégradation protéique est un mélange de peptides ayant un poids moléculaire de 100 à 20 000 Da (déterminé par chromatographie HPLC à perméation de gel) obtenu à partir de la dégradation limitée de la protéine HMG ou de l'amphotérine par une protéase.

6. Utilisation selon l'une quelconque des revendications précédentes, dans laquelle l'agent est combiné avec une boisson ou une nourriture.
